(19) Europäisches Patentamt
European Patent Office
Office européen des brevets

(11) **EP 4 648 063 A1**

(12) **EUROPEAN PATENT APPLICATION**

(43) Date of publication:
**12.11.2025  Bulletin 2025/46**

(21) Application number: **25174682.2**

(22) Date of filing: **07.05.2025**

(51) International Patent Classification (IPC):
***G16H 50/20*** (2018.01)       ***A61B 5/00*** (2006.01)
***G06N 3/08*** (2023.01)

(52) Cooperative Patent Classification (CPC):
**G16H 50/20; A61B 5/7267; G06N 3/045;
G06N 3/08; G06N 3/09**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **09.05.2024  KR 20240061200
20.03.2025  KR 20250035870**

(71) Applicant: **INDUSTRY ACADEMIC COOPERATION
FOUNDATON,
YONSEI UNIVERSITY
Seoul 03722 (KR)**

(72) Inventors:
• **PAK, Hui Nam
03722 Seoul (KR)**
• **KWON, Oh Seok
10494 Goyang (KR)**

(74) Representative: **Müller Schupfner & Partner
Patent- und Rechtsanwaltspartnerschaft mbB
(Muc)
Bavariaring 11
80336 München (DE)**

(54) **METHOD FOR PROVIDING INFORMATION ON SUBJECT AND DEVICE FOR PROVIDING
INFORMATION ON SUBJECT USING THE SAME**

(57)    The present disclosure provides a method for providing information on a subject implemented by a processor, the method including receiving electrocardiogram (ECG) data from the subject, and predicting gender and age of the subject based on the received ECG data using a multi-task learning (MTL)-based prediction model trained to predict the gender and age, using the ECG data as input, in which the prediction model includes a shared layer configured to train a common feature for predicting the gender and age, and a device for providing information using the same.

1000

KIM OO

FEMALE/
61 YEARS OLD

100   300   200

FIG. 1

EP 4 648 063 A1

**Description**

**CROSS-REFERENCE TO RELATED APPLICATIONS**

This application claims the priority of Korean Patent Application No. 10-2024-0061200 filed on May 09, 2024 and No. 10-2025-0035870 filed on Mar 20, 2025 in the Korean Intellectual Property Office, the disclosure of which is incorporated herein by reference.

**BACKGROUND**

**Field**

The present disclosure relates to a method for providing information on a subject and a device for providing information on a subject using the same.

**Description of the Related Art**

Existing medical data analysis models are generally based on a single-task learning (STL) method and are designed to perform only one prediction task.

In this case, individual task learning has the advantage of enabling optimized learning, but since the same data should be repeatedly trained by multiple models, inefficiency in data utilization may occur.

In addition, since an independent learning process is required for each task, computational costs increase, and additional data collection and preprocessing processes are required for individual model optimization. This may make it difficult to apply the single-task learning model in environments where collection is limited, such as medical data.

In particular, for bio-signals such as ECG, there may be a problem that the potential information of the data is not fully utilized when performing single-task learning even though it contains various physiological information.

Accordingly, there is a continuous demand for the development of models that can train a plurality of prediction tasks simultaneously in a single model.

The background technology of the disclosure has been written to facilitate understanding of the present disclosure. It should not be understood that the matters described in the background technology of the disclosure are recognized as prior art.

**SUMMARY**

The inventors of the present disclosure sought to find a new approach for predicting physiological characteristics of the subject by utilizing ECG signals in medical data analysis.

The inventors of the present disclosure have focused on a system for providing information on new subjects by applying a multi-task learning (MTL)-based prediction model.

The inventors of the present disclosure have been able to recognize that data utilization may be increased and computational efficiency may be improved by training a multi-task learning-based prediction model to simultaneously predict the gender and age of a subject from an ECG signal.

In particular, the inventors of the present disclosure have been able to recognize that by applying the shared layers structure to train common features for predicting the gender and age, higher prediction performance may be achieved compared to existing single-task models.

In addition, the inventors of the present disclosure have attempted to balance the loss values since the error is large in age prediction and small in gender prediction.

The inventors of the present disclosure have been able to find that the learning balance between two tasks may be optimized by applying loss weight adjustment and gradient normalization techniques.

In particular, considering that the error in age prediction is large, the inventors of the present disclosure have attempted to adjust the prediction model so that learning of a specific task does not have an excessively dominant effect by applying a logarithmic transformation to a loss function value.

The inventors of the present disclosure have attempted to stabilize prediction model learning by dynamically adjusting the loss weight based on the relative error ratio of each task and appropriately converting high error values occurring in age prediction.

As a result, the inventors of the present disclosure have developed an information provision system using a multi-task learning-based prediction model that may overcome the data utilization inefficiency and computational cost problems of the existing single-task-based prediction method and enable more precise and efficient gender and age prediction using ECG signals.

The inventors of the present disclosure have expected that by providing an information provision system using a prediction model based on a multi-task learning method, the reliability of ECG data analysis may be increased and the usability of a prediction model based on medical data may be expanded.

Furthermore, the inventors of the present disclosure have expected that by providing a new information provision system, the physiological characteristics of a subject in a medical environment may be analyzed more accurately and quickly, and that this may contribute to the development of customized medical services and health monitoring systems in the future.

Accordingly, an object of the present disclosure is to provide a method and device for providing information on a subject using a multi-task learning-based prediction model trained to simultaneously predict gender and age of a subject, using ECG data as input.

The tasks of the present disclosure are not limited to the tasks mentioned above, and other tasks not mentioned will be clearly understood by those skilled in the art from the description below.

In order to solve the above-described problem, a method for providing information on a subject according to one embodiment of the present disclosure is provided.

The method is a method for providing information on a subject implemented by a processor and includes receiving electrocardiogram (ECG) data from the subject and predicting gender and age of the subject based on the received ECG data using a multi-task learning (MTL)-based prediction model trained to predict the gender and age, using the ECG data as input.

In this case, the prediction model may include a shared layer configured to train a common feature for predicting the gender and age.

According to an aspect of the present disclosure, the prediction model may be trained based on a loss ratio-based weight to balance a loss for a task of predicting the gender and age.

According to another aspect of the present disclosure, the loss ratio-based weight may be a ratio of a gender prediction loss value to the sum of the gender prediction loss value and an age prediction loss value, or a ratio of the age prediction loss value to the sum of the gender prediction loss value and the age prediction loss value.

According to still another aspect of the present disclosure, the gender prediction loss value or the age prediction loss value may be a gradient normalized value.

According to still another aspect of the present disclosure, the gender prediction loss value or the age prediction loss value may be a normalized value based on a logarithmic transformation.

According to still another aspect of the present disclosure, the receiving of the ECG data may include receiving a recording paper-based ECG image, and the method may further include extracting a line graph by applying an image processing technique from the recording paper-based ECG image, and converting the extracted ECG signal into data.

According to still another aspect of the present disclosure, the method may further include correcting distortion of the recording paper-based ECG image using object detection.

According to still another aspect of the present disclosure, the converting of the extracted ECG signal into data may further include converting the extracted ECG signal into data according to an XML format.

In order to solve the above-described problem, a device for providing information on a subject according to another embodiment of the present disclosure is provided.

The device includes a communication unit configured to receive electrocardiogram (ECG) data from a subject, and a processor functionally connected to the communication unit.

In this case, the processor is configured to predict gender and age of the subject based on the received ECG data using a multi-task learning (MTL)-based prediction model trained to predict the gender and age, using the ECG data as input.

According to an aspect of the present disclosure, the communication unit may be further configured to receive a recording paper-based ECG image, and the processor may be further configured to extract a line graph by applying an image processing technique from the recording paper-based ECG image, and convert the extracted ECG signal into data.

According to another aspect of the present disclosure, the processor may be further configured to correct distortion of the recording paper-based ECG image using object detection.

According to still another aspect of the present disclosure, the processor may be further configured to convert the extracted ECG signal into data according to an XML format.

In order to solve the above-described problem, a system for providing information on a subject according to still another embodiment of the present disclosure is provided.

The system includes an internal memory storing a multi-task learning (MTL)-based prediction model trained to predict gender and age, by inputting ECG data from the subject and using the ECG data as input, and a processing unit functionally connected to the internal memory.

In this case, the processing unit is configured to predict the gender and age for the subject based on the stored ECG data, using the prediction model, and the prediction model includes a shared layer configured to train common features for predicting the gender and age.

Specific details of other embodiments are included in the detailed description and drawings.

The present disclosure provides a method and device for providing information using a multi-task learning-based prediction model that can simultaneously predict the gender and age of the subject by utilizing ECG data, thereby overcoming the limitations of the existing single-task learning method.

In particular, according to the present disclosure, it is possible to increase data utilization and reduce computational costs by simultaneously predicting the gender and age by applying the multi-task learning-based prediction model. Through this, according to the present disclosure, it is possible to improve learning efficiency and enhance prediction performance by applying a shared layer structure that trains common features.

According to the present disclosure, since the error is large in the case of the age prediction and the error is relatively small in the case of the gender prediction, the problem of learning imbalance between tasks can be solved by applying loss weight adjustment and gradient regularization techniques to balance the loss values.

According to the present disclosure, it is possible to improve the accuracy of a prediction model by improving the quality of input data through a process of correcting distortion that may occur in an ECG recording paper, extracting a line graph by applying an image processing technique, and then converting the extracted line graph into structured data.

That is, the present disclosure improves model learning and inference speed while increasing prediction accuracy compared to existing single-task-based methods, thereby enabling faster analysis in a medical environment.

Through this, the present disclosure can contribute to real-time health monitoring and provision of customized medical services using the ECG data.

Effects according to the present disclosure are not limited to those exemplified above, and further diverse effects are included in the present specification.

The effects of the present disclosure are not limited to the aforementioned effects, and other effects, which are not mentioned above, will be apparently understood to a person having ordinary skill in the art from the following description.

The objects to be achieved by the present disclosure, the means for achieving the objects, and the effects of the present disclosure described above do not specify essential features of the claims, and, thus, the scope of the claims is not limited to the disclosure of the present disclosure.

## BRIEF DESCRIPTION OF DRAWINGS

The above and other aspects, features and other advantages of the present disclosure will be more clearly understood from the following detailed description taken in conjunction with the accompanying drawings, in which:

FIG. 1 is an exemplary diagram illustrating a device-based system for providing information on a subject according to one embodiment of the present disclosure;

FIG. 2A is a block diagram illustrating a configuration of a user device according to one embodiment of the present disclosure;

FIG. 2B is a block diagram illustrating a configuration of a server of a device for providing information according to one embodiment of the present disclosure;

FIGS. 3A to 3F illustrate a procedure of a method for providing information on a subject according to one embodiment of the present disclosure; and

FIGS. 4A to 4C illustrate evaluation results of a prediction model used in a system for providing information on a subject according to various embodiments of the present disclosure.

## DETAILED DESCRIPTION OF THE EMBODIMENT

Hereinafter, the exemplary embodiment of the present disclosure will be described with reference to the accompanying drawings and exemplary embodiments as follows. Scales of components illustrated in the accompanying drawings are different from the real scales for the purpose of description, so that the scales are not limited to those illustrated in the drawings.

The advantages of the disclosure and the method for achieving them will become apparent by referring to the embodiments described in detail below together with the accompanying drawings. However, the present disclosure is not limited to the embodiments disclosed below, but may be implemented in various different forms, and these embodiments are provided only to make the disclosure of the present disclosure complete and to fully inform those skilled in the art of the scope of the disclosure.

The shapes, sizes, ratios, angles, numbers, or the like disclosed in the drawings for explaining embodiments of the present disclosure are exemplary, and therefore, the present disclosure is not limited to the matters illustrated. In addition, when describing the present disclosure, when it is determined that a detailed description of a related known technology may unnecessarily obscure the gist of the present disclosure, the detailed description will be omitted. When the terms "include", "have", "consist of", or the like are used in the present specification, other parts may be added unless "only" is used. When a component is expressed in singular, it includes a case where the plural is included unless there is a specifically explicit description.

When interpreting components, it is interpreted as including the error range even if there is no separate explicit description.

The individual features of the various embodiments of the present disclosure may be partially or wholly coupled or combined with each other, and as can be fully understood by those skilled in the art, various technical connections and operations are possible, and each embodiment may be implemented independently of each other or may be implemented together in a related relationship.

For clarity in the interpretation of the present specification, the terms used in the present specification are defined below.

The term "subject" used in the present specification may mean a target that provides ECG data and whose gender or age is to be predicted.

The term "electrocardiogram (ECG) data" as used in the present specification may mean electrophysiological signals that record the cardiac activity of the subject. The ECG data may be used to analyze the heart rhythm, heart rate, and cardiac health status of a subject, and may be applied to a multi-task learning-based prediction model for predicting physiological information of the subject.

The term "multi-task learning-based prediction model" as used in the present specification may mean an artificial neural network model trained to simultaneously predict gender and age.

In various embodiments of the present disclosure, the multi-task learning-based prediction model may operate in a manner of training common features including a shared layer, and then generating respective outputs through an individual fully connected layer (FC layer) for gender and age prediction. However, the structure thereof is not limited to the above, and the multi-task learning-based prediction model may include various neural network structures.

For example, the multi-task learning-based prediction model may be configured to effectively train time-series features of ECG signals by applying a neural network structure based on a residual neural network (ResNet). In this case, the ResNet structure can prevent a gradient vanishing problem that may occur in the neural network by including residual connections, and improve the feature extraction performance for gender and age prediction.

However, the present disclosure is not limited to this, and the multi-task learning-based prediction model may also include more diverse neural network structures.

For example, various deep learning models such as DenseNet, EfficientNet, Transformer-based models, or CNN-RNN hybrid structures may be applied as the multi-task learning-based prediction model. In addition, the multi-task learning-based prediction model may be implemented as a long short-term memory (LSTM), a gated recurrent unit (GRU), or a 1-dimensional convolutional neural network (1D-CNN)-based model to more precisely analyze the time series pattern of the ECG signal.

Meanwhile, in various embodiments of the present disclosure, the prediction model may be expanded into a model that performs multiple predictions, such as health status analysis, prediction of the probability of occurrence of a specific disease, and recommendation of personalized medical services, in addition to age and gender prediction, when there is sufficient training data for the subject.

The term "shared layer" as used in the present specification may mean a neural network layer that trains common features for gender and age prediction in the multi-task learning-based prediction model.

In various embodiments of the present disclosure, the shared layer may be configured to extract common features based on the input ECG data and share the common features in the gender and age prediction task. However, the present disclosure is not limited thereto, and the shared layer may be implemented in various network structures.

The term "loss ratio-based weight" as used in the present specification may mean a method of adjusting a learning weight based on a ratio of a specific task loss value to the sum of a gender prediction loss value and an age prediction loss value.

For example, the loss ratio-based weight may be defined as the following [Mathematical Formula 1].

[Mathematical Formula 1]

$$\omega_{age} = \frac{L_{age}}{(L_{age} + L_{gender})}$$

$$\omega_{gender} = \frac{L_{gender}}{(L_{age} + L_{gender})}$$

Here, $\omega_{age}$ may mean a weight for age prediction, $\omega_{gender}$ may mean a weight for gender prediction, $L_{age}$ may mean a loss value for age prediction, and $L_{gender}$ may mean a loss value for gender prediction.

However, the application of weight in learning a predictive model is not limited to this.

The term "gradient normalization" as used in the present specification may mean a method of preventing a gradient value of a specific task from becoming excessively large and ensuring balanced learning between gender and age prediction tasks.

The term "logarithmic transformation" as used in the present specification may mean a regularization technique that reduces the difference in scale of loss function values so that the loss values of a specific task do not have an excessive influence on model learning.

In various embodiments of the present disclosure, a logarithmic transformation technique of the loss value may be applied to reduce the difference in the scale of the loss function value and induce stable learning, and normalization may be performed according to the following [Mathematical Formula 2].

[Mathematical formula 2]

$$L_{age} = \log(L_{age} + 1)$$

$$L_{gender} = \log(L_{gender} + 1)$$

Meanwhile, in more diverse embodiments of the present disclosure, the total loss function may be defined by the following [Mathematical Formula 3].

[Mathematical Formula 3]

$$L_{total} = N_{grad} \times \omega_{age} \times L_{age} + N_{grad} \times \omega_{gender} \times L_{gender}$$

Here, $L_{total}$ is a total loss function, $N_{grad}$ may mean a gradient normalization factor, and each of $\omega_{age}$, $\omega_{gender}$, $L_{age}$, and $L_{gender}$ may be determined as the ratio of each loss value to the sum of loss values as described above, or may be determined by the logarithmic transformation or gradient normalization.

This learning method may contribute to maintaining the learning balance even when the loss value of a specific task is large or small, and to stably train the model so that gender and age prediction can be optimized simultaneously.

The term "recording paper-based ECG image" as used in the present specification may mean data in which the ECG signal is provided in the form of a paper record.

In various embodiments of the present disclosure, the recording paper-based ECG image may be converted into digital data after extracting the signal through the image processing technique.

The term "object detection" as used in the present specification may mean an image processing technique applied to correct distortion of the recording paper-based ECG image.

According to more diverse embodiments, the location of an ECG signal in a recording paper may be recognized based on the object detection, and signal distortion may be corrected by applying the image processing technique. In addition, a line graph may be extracted based on the recognized signal, and may be converted into a structured data format such as an XML format in the process of converting the extracted line graph into data.

This approach allows accurate signal extraction even when ECG recording papers are taken in various environments or contain distortion, providing reliable data to the prediction model.

The term "performing conversion into data according to an XML format" as used in the present specification may refer to a method of converting and storing ECG signals into a structured data format.

In further embodiments of the present disclosure, time and amplitude data of the extracted ECG signal may be converted according to the XML format.

However, the present disclosure is not limited to this, and the extracted ECG signal may also be converted into other formats such as JSON, CSV, or the like.

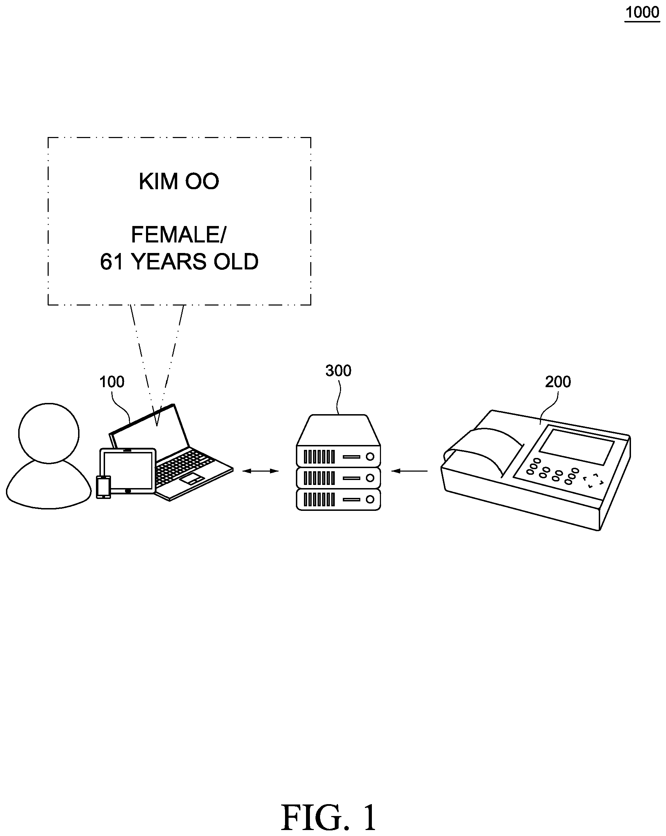

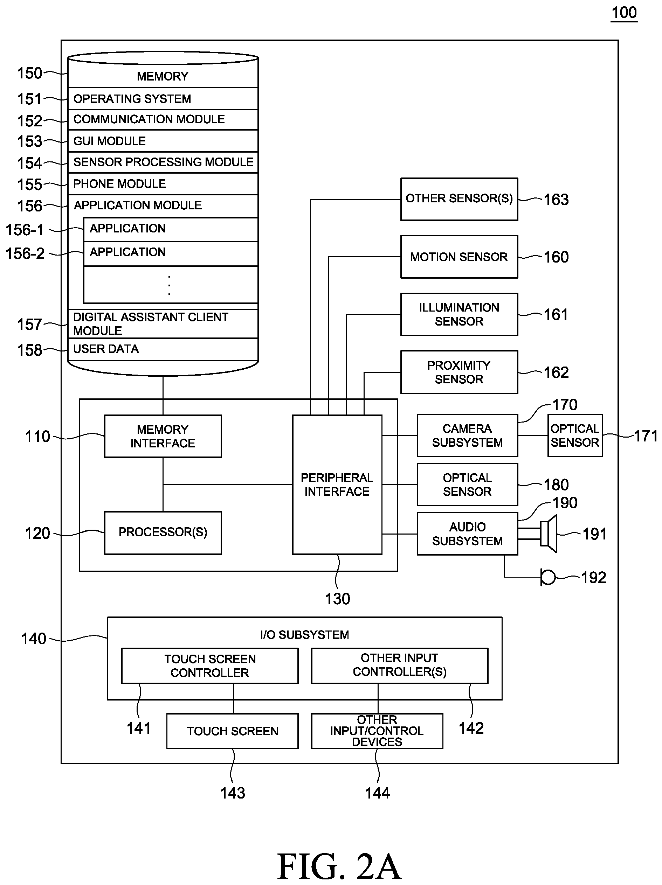

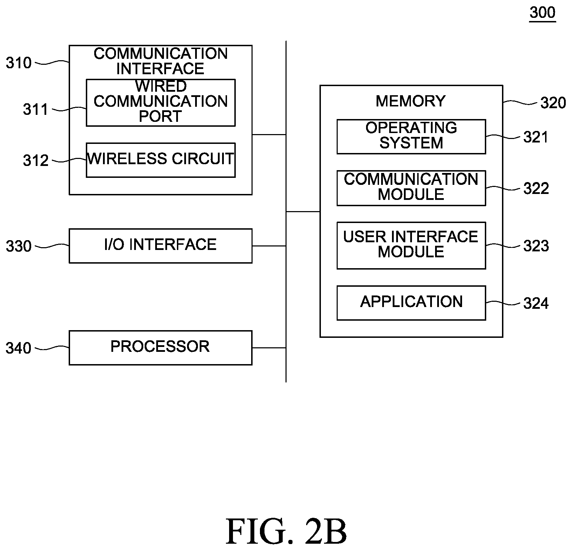

Hereinafter, with reference to FIGS. 1 and 2A and 2B, a system for providing information on a subject using a device for providing information on a subject, a user device, and the device for providing information on a subject according to one embodiment of the present disclosure will be described.

FIG. 1 illustrates the system for providing information on a subject using the device for providing information on a subject according to one embodiment of the present disclosure. FIG. 2A is a block diagram illustrating a configuration of the user device according to one embodiment of the present disclosure. FIG. 2B is a block diagram illustrating a configuration of a server of the device for providing information according to one embodiment of the present disclosure.

First, referring to FIG. 1, an information provision system 1000 may be a system configured to provide information on a subject. In this case, the information provision system 1000 may be configured with a user device 100 that receives information on the subject, for example, gender and age prediction information using ECG data, an ECG data collection device 200 that provides the ECG data, and an information providing server 300 that generates information on the subject based on the received ECG data.

First, the user device 100 is an electronic device that provides a user interface for displaying information on the subject, and may include at least one of a smartphone, a tablet personal computer (PC), a laptop, and/or a PC.

The user device 100 may receive information on the subject from the information providing server 300, for example, predicted gender and age information based on the ECG data, and display the received information through a display unit.

The ECG data collection device 200 is a device that acquires the ECG data of the subject, and may include a device that outputs the ECG signal in the form of a recording paper or a medical device that collects the signals in digital form. In addition, when recording paper-based ECG data is provided, an image processing technique may be applied to convert the ECG data into digital data.

The information providing server 300 may be a server that performs an operation to predict the gender and age of the subject using the multi-task learning-based prediction model from the ECG data provided from the ECG data collection device 200. To this end, the information providing server 300 may analyze the features of the ECG data using a prediction model including a ResNet-based neural network and generate gender and age prediction information.

In addition, the information providing server 300 may be a device for accessing a web server providing a web page or a mobile web server providing a mobile website, but is not limited thereto. Through this, the user device 100 may support to access the information providing server 300 through the Internet and check the gender and age information of the subject in real time.

Next, components of the information providing server 300 of the present disclosure will be specifically described with reference to FIGS. 2A and 2B.

First, referring to FIG. 2A, the user device 100 may include a memory interface 110, one or more processors 120, and a peripheral interface 130. Various components within the user device 100 may be connected by one or more communication buses or signal lines.

The memory interface 110 is connected to a memory 150 and may transmit various data to the processor 120. Here, the memory 150 may include at least one type of storage medium among flash memory type, hard disk type, multimedia card micro type, card type memory (for example, SD or XD memory, or the like), RAM, SRAM, ROM, EEPROM, and PROM, network storage, cloud, and blockchain data.

In various embodiments, the memory 150 may store at least one of an operating system 151, a communication module 152, a graphical user interface (GUI) module 153, a sensor processing module 154, a telephone module 155, and an application module 156. Specifically, the operating system 151 may include instructions for processing basic system services and instructions for performing hardware operations. The communication module 152 may communicate with at least one of one or more other devices, computers, and servers. The graphical user interface module (GUI) 153 may process a graphical user interface. The sensor processing module 154 may process sensor-related functions (for example, processing voice input received using one or more microphones 192). The telephone module 155 may process telephone-related functions. The application module 156 may perform various functions of the user application, such as electronic messaging, web browsing, media processing, navigation, imaging, and other processing functions. In addition, the user device 100 may store one or more software applications 156-1 and 156-2 associated with one type of service in the memory 150.

In various embodiments, the memory 150 may store a digital assistant client module 157 (hereinafter, DA client module), and thus store instructions for performing client-side functions of the digital assistant and various user data 158.

Meanwhile, the DA client module 157 may obtain the user's voice input, text input, touch input, and/or gesture input through various user interfaces (for example, I/O subsystem 140) provided in the user device 100.

Additionally, the DA client module 157 can output data in audiovisual and tactile forms. For example, the DA client module 157 may output data consisting of a combination of at least two or more of voice, sound, notification, text message, menu, graphic, video, animation, and vibration. Additionally, the DA client module 157 may communicate with a digital assistant server (not illustrated) using the communication subsystem 180.

In various embodiments, the DA client module 157 may collect additional information on the surroundings of the user device 100 from various sensors, subsystems, and peripheral devices to construct a context associated with the user input. For example, the DA client module 157 may provide context information along with the user input to a digital assistant server to infer the user's intent. Here, the context information that may accompany the user input may include sensor information, such as lighting, ambient noise, ambient temperature, images of the surrounding environment, video, or the like. As another example, the context information may include the physical state of the user device 100 (for example, device orientation, device position, device temperature, power level, speed, acceleration, motion pattern, cellular signal strength, or the like). As another example, the context information may include information (for example, processes running on the user device 100, installed programs, past and current network activity, background services, error logs, resource usage, or the like) related to the software state of the user device 100.

In various embodiments, the memory 150 may include additional or deleted instructions, and further may include additional configurations other than those illustrated in FIG. 2A of the user device 100, or may exclude some configurations.

The processor 120 may control the overall operation of the user device 100 and execute various commands to implement an interface that provides information on the subject, for example, subject information, by driving an application or program stored in the memory 150.

The processor 120 may correspond to a computational device such as a central processing unit (CPU) or an application processor (AP). In addition, the processor 120 may be implemented in the form of an integrated chip (IC) such as a system on chip (SoC) in which various computational devices such as a neural processing unit (NPU) are integrated.

The peripheral interface 130 may be connected to various sensors, subsystems, and peripheral devices to provide data so that the user device 100 can perform various functions. Here, it can be understood that the user device 100 performs a certain function as being performed by the processor 120.

The peripheral interface 130 may receive data from a motion sensor 160, a light sensor (illumination sensor) 161, and a proximity sensor 162, through which the user device 100 may perform orientation, light, and proximity detection functions, or the like. For another example, the peripheral interface 130 may receive data from other sensors 163 (positioning system-GPS receiver, temperature sensor, biometric sensor), through which the user device 100 may perform functions related to the other sensors 163.

In various embodiments, the user device 100 may include a camera subsystem 170 connected to a peripheral interface 130 and an optical sensor 171 connected thereto, which may enable the user device 100 to perform various photographic functions, such as taking pictures and recording video clips.

In various embodiments, the user device 100 may include the communication subsystem 180 connected to the peripheral interface 130. The communication subsystem 180 may include one or more wired/wireless networks and may include various communication ports, radio frequency transceivers, and optical transceivers.

In various embodiments, the user device 100 includes an audio subsystem 190 connected to the peripheral interface 130, the audio subsystem 190 including one or more speakers 191 and one or more microphones 192, such that the user device 100 may perform voice-activated functions, such as voice recognition, voice replication, digital recording, and telephony.

In various embodiments, the user device 100 may include the I/O subsystem 140 connected to a peripheral interface 130. For example, the I/O subsystem 140 may control a touch screen 143 included in the user device 100 via a touch screen controller 141. As an example, the touch screen controller 141 may detect a user's contact and movement or cessation of contact and movement using any one of a plurality of touch sensing technologies, such as capacitive, resistive, infrared, surface acoustic wave technology, proximity sensor array, and the like. As another example, the I/O subsystem 140 may control other input/control devices 144 included in the user device 100 via other input controller(s) 142. As an example, the other input controller(s) 142 may control one or more buttons, rocker switches, thumb-wheels, infrared ports, USB ports, and pointer devices such as a stylus.

Next, referring to FIG. 2B, the information providing server 300 may include a communication interface 310, a memory 320, an I/O interface 330, and a processor 340, each component of which may communicate with each other through one or more communication buses or signal lines.

The communication interface 310 may be connected to the user device 100 and the ECG data collection device 200 via a wired/wireless communication network to exchange data. For example, the communication interface 310 may transmit the information (or a file) on the subject to the user device 100.

Meanwhile, the communication interface 310 that enables transmission and reception of such data includes a communication port 311 and a wireless circuit 312, in which the wired communication port 311 may include one or more wired interfaces, for example, Ethernet, universal serial bus (USB), FireWire, or the like. In addition, the wireless circuit 312 may transmit and receive data with an external device via an RF signal or an optical signal. In addition, the wireless circuit 312 may use at least one of a plurality of communication standards, protocols, and technologies, for example, GSM, EDGE, CDMA, TDMA, Bluetooth, Wi-Fi, VoIP, Wi-MAX, or any other suitable communication protocol.

The memory 320 may store various data used in the information providing server 300. For example, the memory 320 may be configured to store prediction models, data generated while processing the prediction models, learning parameters of the model, and information on the subject, such as age and gender.

In various embodiments, the memory 320 may include a volatile or nonvolatile storage medium capable of storing various data, commands, and information. For example, the memory 320 may include at least one type of storage medium among a flash memory type, a hard disk type, a multimedia card micro type, a card type memory (for example, an SD or XD memory, or the like), a RAM, an SRAM, a ROM, an EEPROM, a PROM, a network storage, a cloud, and blockchain data.

In various embodiments, the memory 320 may store a configuration of at least one of an operating system 321, a communication module 322, a user interface module 323, and one or more applications 324.

The operating system 321 (for example, an embedded operating system such as LINUX, UNIX, MAC OS, WINDOWS, VxWorks, or the like) may include various software components and drivers to control and manage general system operations (for example, memory management, storage device control, power management, or the like) and may support communication between various hardware, firmware, and software components.

The communication module 322 may support communication with other devices through the communication interface 310. The communication module 322 may include various software components for processing data received by the wired communication port 311 or wireless circuit 312 of the communication interface 310.

The user interface module 323 may receive a user's request or input from a keyboard, touch screen, microphone, or the like through the I/O interface 330 and provide the user interface on the display.

The application 324 may include a program or module configured to be executed by one or more processors 340. Here, the application for providing the information on the subject may be implemented on a server farm.

The I/O interface 330 may connect an input/output device (not illustrated) of the information providing server 300, such as at least one of a display, a keyboard, a touch screen, and a microphone, to the user interface module 323. The I/O interface 330 may receive user input (for example, voice input, keyboard input, touch input, or the like) together with the user interface module 323 and process a command according to the received input.

The processor 340 is connected to the communication interface 310, the memory 320, and the I/O interface 330 to control the overall operation of the information providing server 300, and may perform various commands for providing information through an application or program stored in the memory 320.

The processor 340 may correspond to a computational device such as a central processing unit (CPU) or an application processor (AP). In addition, the processor 340 may be implemented in the form of an integrated chip (IC) such as a system on chip (SoC) in which various computational devices are integrated. Alternatively, the processor 340 may include a module for calculating an artificial neural network model such as a neural processing unit (NPU).

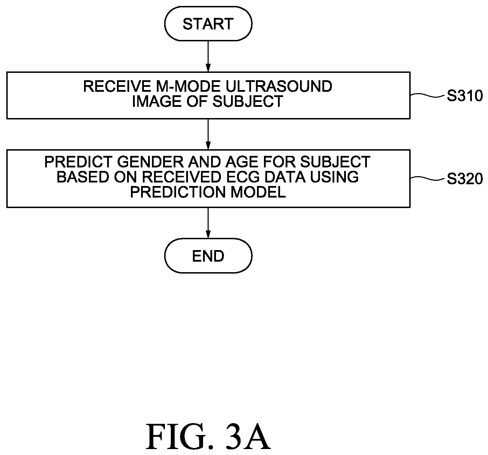

Hereinafter, a method for providing information on a subject according to various embodiments of the present disclosure will be specifically described with reference to FIGS. 3A to 3F.

FIGS. 3A to 3F illustrate a procedure of the method for providing information on a subject according to one embodiment of the present disclosure.

First, referring to FIG. 3A, the information provision procedure according to one embodiment of the present disclosure is as follows. First, the ECG data of the subject is received (S310). Then, the gender and age of the subject are each predicted through the prediction model using the received ECG data (S320).

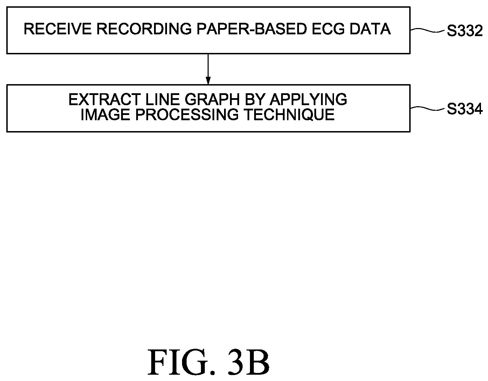

Referring to FIG. 3B together, in various embodiments of the present disclosure, recording paper-based ECG data is received (S332) and a line graph is extracted from the received ECG data by applying an image processing technique (S334). Through this, the recording paper-based ECG data may be converted into a digital signal and utilized as the input data for the prediction model.

According to an aspect of the present disclosure, correction for distortion of the recording paper-based ECG image may be performed using the object detection.

For example, when the ECG recording paper is tilted or deformed during the shooting process, the location of the ECG signal may be recognized through the object detection model, and a signal alignment algorithm may be applied to realign the ECG recording paper to the reference axis. In addition, a method of removing the background lines of the graph paper and optimizing the contrast of the signal may be applied to extract the graph more clearly. The present disclosure is not limited thereto, and various image processing techniques can be additionally applied.

According to another feature of the present disclosure, the extracted ECG signal may be converted into data according to the XML format.

For example, the extracted ECG signal may be saved as an XML file by structuring the time-voltage data for each lead, and the main features (for example, amplitude and duration of P wave, T wave, or the like) of the signal may be included in the form of tags. In addition, additional metadata (for example, recording time, lead type, sampling rate, or the like) for the ECG analysis may be saved together. However, the present disclosure is not limited, and the extracted ECG signal may also be converted into data formats such as JSON and CSV

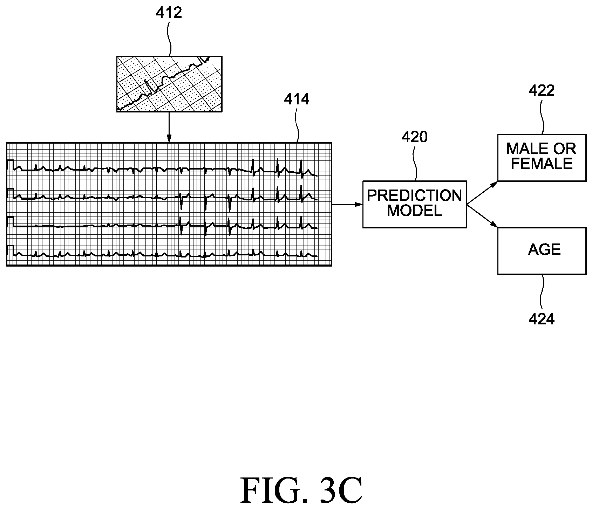

Referring to FIG. 3C together, a recording paper-based ECG image 412 is converted into data through a process in which the line graph is extracted through an image processing technique and converted into digital data 414, and this data may be used as input data for the prediction model 420.

The prediction model 420 may predict the gender 422 and age 424 of the subject by analyzing the ECG data 414. In this case, the prediction model 420 is trained based on multi-task learning, and loss ratio-based weight adjustment, gradient regularization, and logarithmic transformation techniques may be applied to balance the gender and age prediction tasks.

Meanwhile, in more diverse embodiments of the present disclosure, the prediction model 420 may include a shared layer.

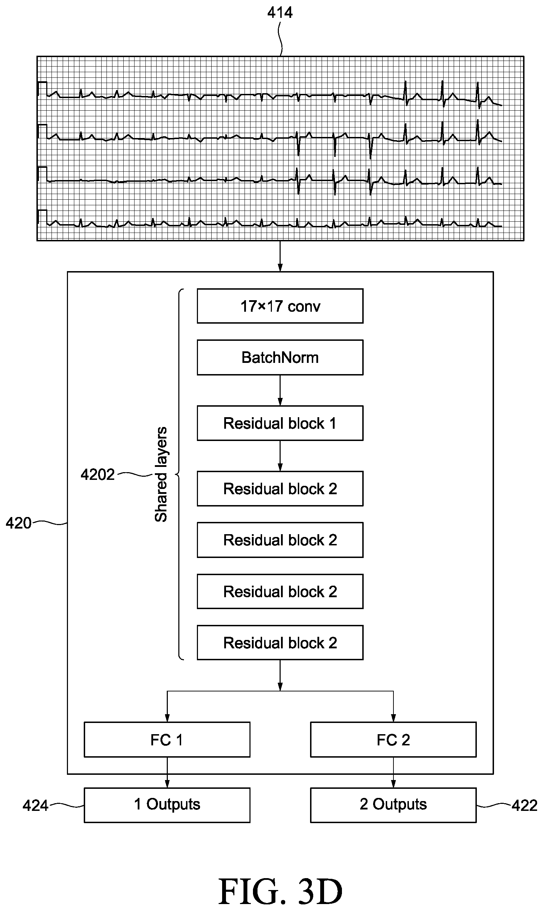

More specifically, referring to FIG. 3D together, when the ECG data 414 is input into the prediction model 420, the prediction model 420 may extract initial features by applying a convolutional layer and a batch normalization (BatchNorm) layer. Thereafter, the main features of the ECG signal are extracted through a deep neural network including a residual block 1 and a plurality of residual blocks 2.

In this case, the shared layer 4202 is configured to train common features for predicting the gender and age, and in this process, effective learning can be possible even in a deep network structure by utilizing ResNet-based residual connections.

After passing through the shared layer 4202, the output for prediction of the gender 422 and age 424 of the subject is performed through the output layers of a fully connected layer 1 (FC1) and a fully connected layer 2 (FC2).

According to an aspect of the present disclosure, the learning balance between the gender and age prediction tasks is maintained through the model structure including a shared layer 4202, and the learning stability of the model may be improved by applying the loss ratio-based weight adjustment and gradient regularization technique, and the details on learning of the prediction model will be described later.

Through this structure, the prediction model 420 of the present disclosure may simultaneously predict the gender and age of the subject with high reliability based on the ECG data, and may provide more efficient and precise results compared to existing single-task learning models.

Meanwhile, the structure of the prediction model 420 is not limited to this.

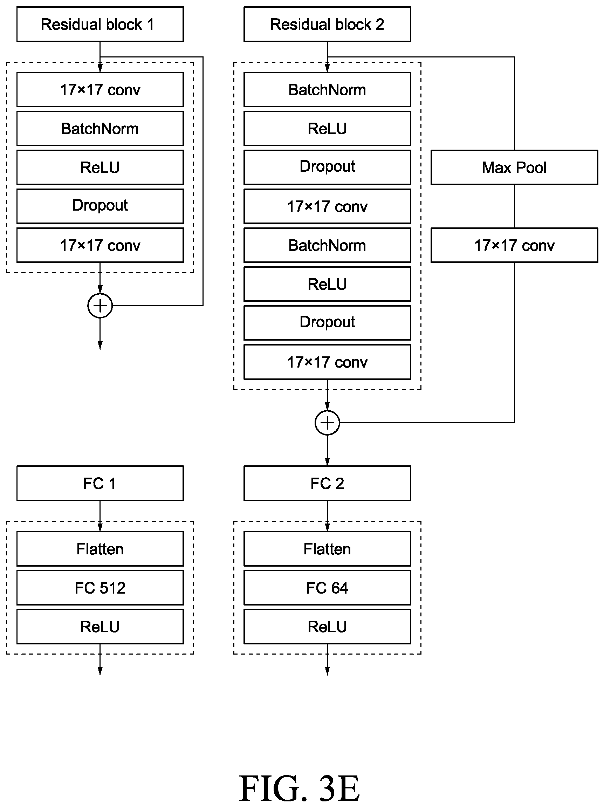

For example, referring further to FIG. 3E, the prediction model 420 has a deep neural network structure including the residual block 1 and residual block 2, and the residual block 1 may include layers such as a $17 \times 17$ convolutional layer ($17 \times 17$ conv), batch normalization (BatchNorm), ReLU activation function, and dropout. In this case, information loss may be reduced through a residual connection (skip connection) that maintains the same input.

Meanwhile, the residual block 2 includes two $17 \times 17$ convolutional layers, and may finally include the residual connection after applying the batch normalization, ReLU activation function, and dropout, respectively.

Additionally, max pool and additional $17 \times 17$ convolutional layers may be applied in specific layers, allowing the model to train features with more diverse resolutions.

Meanwhile, the prediction model 420 may predict gender FC 1 and age FC 2, respectively, through the FC layer after converting the feature map into a one-dimensional vector by including a flatten layer in the output stage.

In this way, the prediction model 420 may include a configuration of various residual blocks and FC layers, and may perform more precise gender and age prediction by adjusting the depth of the neural network.

In various embodiments of the present disclosure, the structure of the prediction model may be combined with various deep learning networks, and may also be extended by combining with a transformer-based model or an RNN-based network.

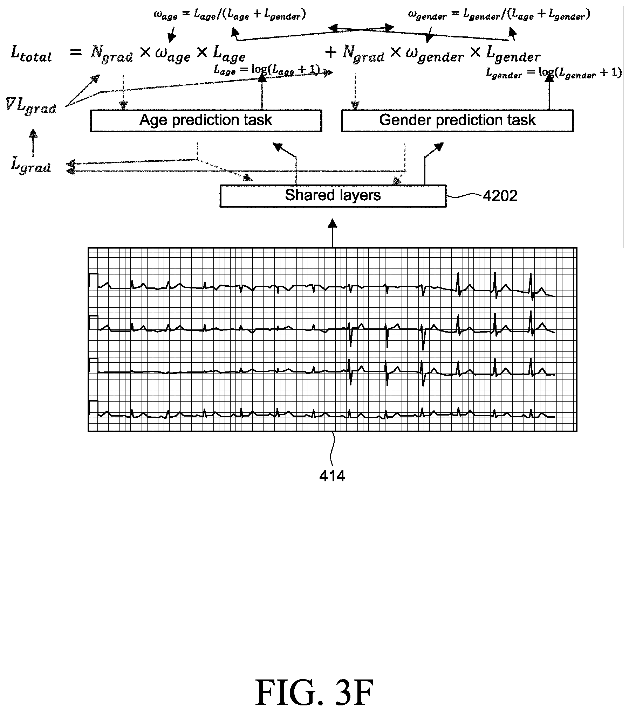

Below, learning for the prediction model used in various embodiments of the present disclosure is specifically described with reference to FIG. 3F.

Referring to FIG. 3F, the recording paper-based ECG data 414 is provided as input to the prediction model 420, and features may be extracted through a neural network including the shared layer 4202.

According to various embodiments of the present disclosure, the prediction model may apply a multi-task learning structure so as to perform gender and age prediction simultaneously, and for this purpose, weight adjustment and gradient regularization techniques of a loss function may be applied.

More specifically, the total loss function is adjusted to maintain the balance between gender and age prediction tasks, which may be defined as the following [Mathematical Formula 3].

[Mathematical Formula 3]

$$L_{total} = N_{grad} \times \omega_{age} \times L_{age} + N_{grad} \times \omega_{gender} \times L_{gender}$$

$N_{gard}$ may mean a gradient normalization factor, which may be adjusted to prevent the loss of a specific task from having an excessive influence on the learning process.

$\omega_{age}$ may mean the weight for age prediction, $\omega_{gender}$ may mean the weight for gender prediction, and the loss ratio-based weight may be defined by the following [Mathematical Formula 1].

[Mathematical Formula 1]

$$\omega_{age} = \frac{L_{age}}{(L_{age} + L_{gender})}$$

$$\omega_{gender} = \frac{L_{gender}}{(L_{age} + L_{gender})}$$

$L_{age}$ may mean the loss value for age prediction, and $L_{gender}$ may mean the loss value for gender prediction, and may be based on the logarithmic transformation of [Mathematical Formula 2] below.

[Mathematical Formula 2]

$$L_{age} = \log(L_{age} + 1)$$

$$L_{gender} = \log(L_{gender} + 1)$$

Learning a prediction model in this way may contribute to maintaining the loss balance so that a specific task is not over-trained. In particular, since the error is relatively large in the age prediction task, the logarithmic transformation may be applied to ensure the stability of training the prediction model.

That is, according to the present disclosure, it is possible to improve learning efficiency and prediction accuracy by providing the multi-task learning-based prediction model that simultaneously predicts the gender and age of the subject based on the ECG data.

The prediction model of the present disclosure trains the common features for predicting the gender and age by including the shared layer, thereby reducing the computational cost of the model and increasing data utilization compared to the existing learning method for each individual task.

In addition, the present disclosure may prevent the problem of the loss value of a specific task being excessively influenced by applying loss balancing and gradient regularization techniques, and secure the stability of learning by applying logarithmic transformation to correct the high error of the age prediction task.

Through this, the present disclosure may resolve learning imbalances between the gender and age predictions, provide more precise prediction performance, and contribute to increasing the reliability of real-time health monitoring and personalized medical services using the ECG data.

**Evaluation Example: Evaluation of Prediction Model Applied to Various Embodiments of Present disclosure**

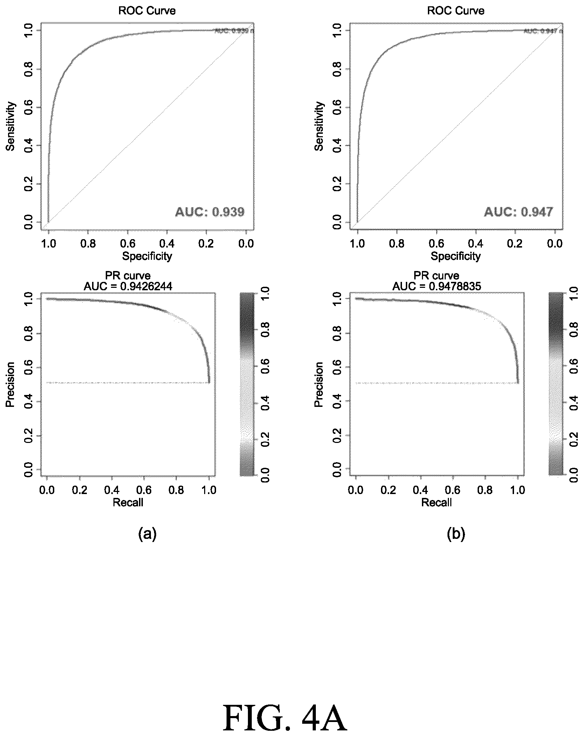

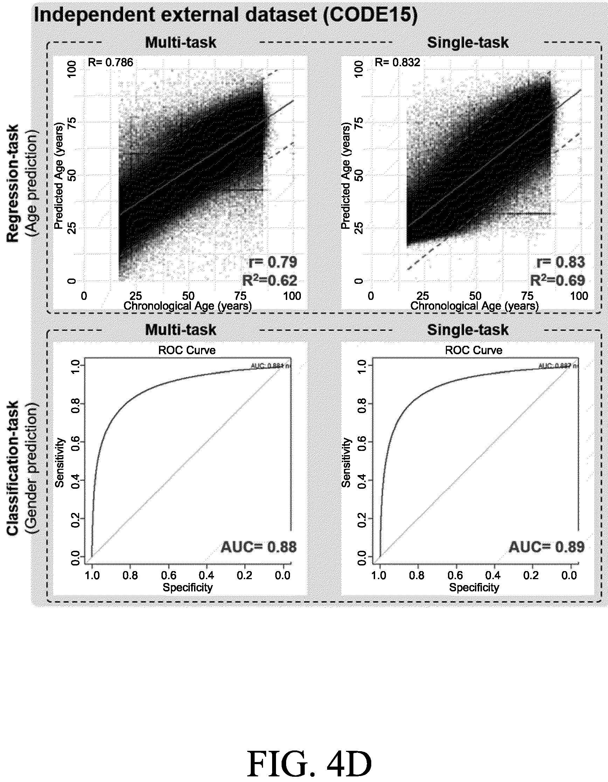

In the following examples, the evaluation results of the prediction model used in an information provision system according to various embodiments of the present disclosure are described with reference to FIGS. 4A to 4C.

First, referring to FIG. 4A, the results of comparing the gender task performance of the multi-task learning model and the single-task learning model are illustrated.

More specifically, the area under the curve (ACU) value of the multi-task learning model was measured to be 0.939, and the AUC value of the single-task learning model was measured to be 0.947, indicating that the two models have similar levels of classification performance in the gender prediction task.

In addition, the precision-recall (PR) curves of the two models illustrate similar trends, which may imply that effective prediction is possible without performance degradation in the gender prediction task even when the multi-task learning structure is applied.

In other words, the above results suggest that the multi-task learning-based prediction model of the present disclosure may perform the multi-task learning without performance degradation compared to the single-task learning model.

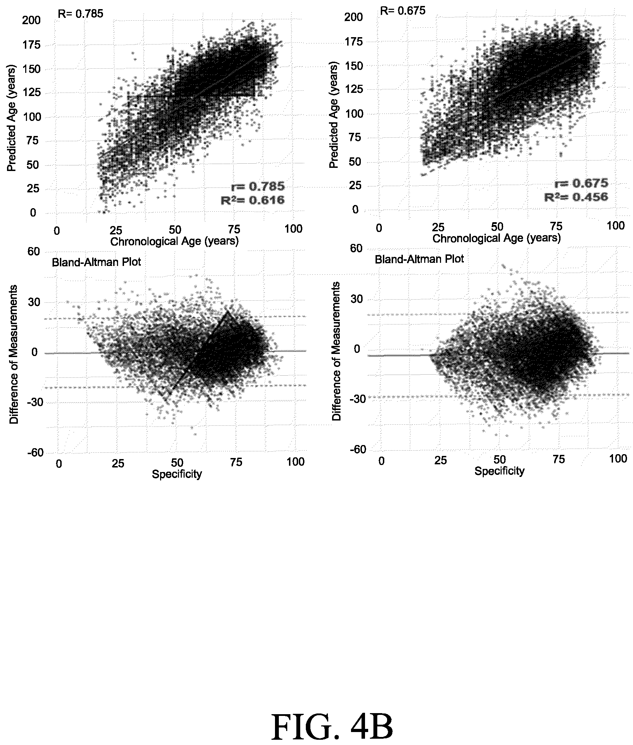

Next, referring to FIG. 4B, the results of comparing the age regression performance of the multi-task learning model and the single-task learning model are illustrated.

More specifically, a correlation coefficient (r) value of the multi-task learning model was 0.785 and the determination coefficient ($R^2$) value was 0.616, which means that it maintains high accuracy in the age prediction task.

On the other hand, for the single-task learning model, the correlation coefficient (r) value was 0.675 and the determination coefficient ($R^2$) value was 0.456, indicating lower performance than the multi-task learning model.

Referring to the Bland-Altman analysis results, the multi-task learning model illustrates relatively high consistency between the predicted value and actual value, and the variance tends to be distributed smaller. This suggests that the multi-task learning model may contribute to improving the performance of age prediction while maintaining learning balance between the gender and age tasks.

That is, the above results may imply that the prediction model of the present disclosure has improved age prediction performance compared to the single-task learning model, and may provide more precise results through loss balance adjustment, especially in tasks with large errors.

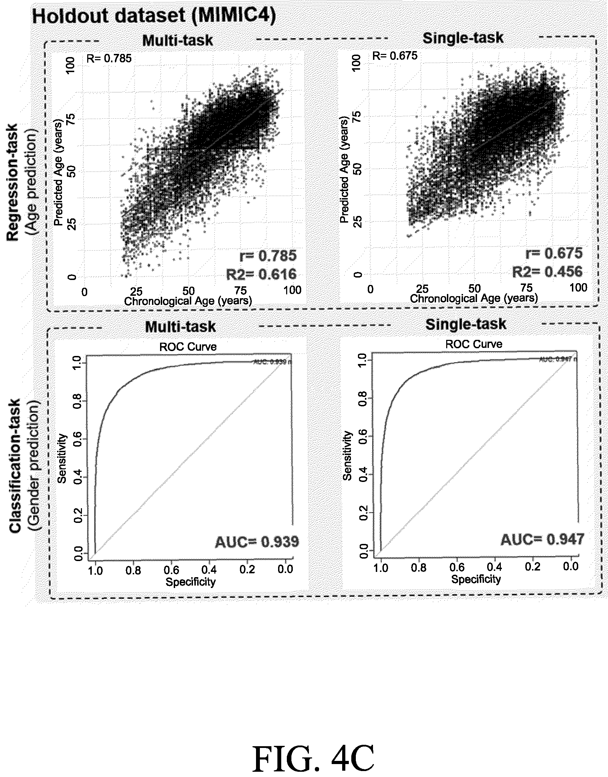

Next, referring to FIG. 4C, the performance comparison results of the multi-task learning model and the single-task learning model for an independent external dataset CODE15 are illustrated.

In this case, the performance difference between the two models was not significant in the gender prediction task.

More specifically, in the age prediction (regression) task, the correlation coefficient value of the multi-task learning model was measured as 0.79 and the determination coefficient value was 0.62, while for the single-task learning model, the correlation coefficient value was 0.83 and the determination coefficient value was 0.69.

Meanwhile, in the gender prediction task, the AUC value of the multi-task learning model was measured to be 0.88, and the AUC value of the single-task learning model was measured to be 0.89, indicating that there is not a large difference in performance between the two models.

These results may imply that the prediction model of the present disclosure maintains the gender prediction performance compared to a single-task learning model.

That is, according to the present disclosure, it is possible to improve learning efficiency and prediction accuracy by providing the multi-task learning-based prediction model that simultaneously predicts the gender and age of the subject based on the ECG data.

The prediction model of the present disclosure trains the common features for predicting the gender and age by including the shared layer, thereby reducing the computational cost of the model and increasing data utilization compared to the existing learning method for each individual task.

**In** addition, the present disclosure may prevent the problem of the loss value of a specific task being excessively influenced by applying loss balancing and gradient regularization techniques, and secure the stability of learning by applying logarithmic transformation to correct the high error of the age prediction task.

Through this, the present disclosure may resolve learning imbalances between the gender and age predictions, provide more precise prediction performance, and contribute to increasing the reliability of real-time health monitoring and personalized medical services using the ECG data.

Although the embodiments of the present disclosure have been described in more detail with reference to the attached drawings, the present disclosure is not necessarily limited to these embodiments, and various modifications may be made without departing from the technical idea of the present disclosure. Accordingly, the embodiments disclosed in the present disclosure are not intended to limit the technical idea of the present disclosure, but to explain it, and the scope of the technical idea of the present disclosure is not limited by these embodiments. Therefore, it should be understood that the embodiments described above are exemplary in all aspects and not restrictive. The protection scope of the present disclosure should be interpreted by the following claims, and all technical ideas within a scope equivalent thereto should be interpreted as being included in the scope of the rights of the present disclosure.

**Claims**

1. A method for providing information on a subject implemented by a processor, the method comprising:

   receiving electrocardiogram (ECG) data from the subject; and
   predicting gender and age of the subject based on the received ECG data using a multi-task learning (MTL)-based prediction model trained to predict the gender and age, using the ECG data as input,
   wherein the prediction model includes a shared layer configured to train a common feature for predicting the gender and age.

2. The method according to claim 1, wherein the prediction model is a model trained based on a loss ratio-based weight to balance a loss for a task of predicting the gender and age.

3. The method according to claim 2, wherein the loss ratio-based weight is a ratio of a gender prediction loss value to a sum of the gender prediction loss value and an age prediction loss value, or a ratio of the age prediction loss value to the sum of the gender prediction loss value and the age prediction loss value.

4. The method according to claim 3, wherein the gender prediction loss value or the age prediction loss value is a gradient normalized value.

5. The method according to claim 3, wherein the gender prediction loss value or the age prediction loss value is a normalized value based on logarithmic transformation.

6. The method according to claim 1, wherein the receiving of the ECG data includes receiving a recording paper-based ECG image,

   the method further includes
   extracting a line graph by applying an image processing technique from the recording paper-based ECG image, and
   converting the extracted ECG signal into data.

7. The method according to claim 6, further comprising correcting distortion of the recording paper-based ECG image using object detection.

8. The method according to claim 6, wherein the converting of the extracted ECG signal into data includes converting the extracted ECG signal into data according to an XML format.

9. A device for providing information on a subject, the device comprising:

a communication unit configured to receive electrocardiogram (ECG) data from a subject; and
a processor functionally connected to the communication unit,
wherein the processor is configured to predict gender and age of the subject based on the received ECG data using a multi-task learning (MTL)-based prediction model trained to predict the gender and age, using the ECG data as input, and
the prediction model includes a shared layer configured to train a common feature for predicting the gender and age.

10. The device according to claim 9, wherein the prediction model is a model trained based on a loss ratio-based weight to balance a loss for a task of predicting the gender and age.

11. The device according to claim 10, wherein the loss ratio-based weight is a ratio of a gender prediction loss value to a sum of the gender prediction loss value and an age prediction loss value, or a ratio of the age prediction loss value to the sum of the gender prediction loss value and the age prediction loss value.

12. The device according to claim 11, wherein the gender prediction loss value or the age prediction loss value is a gradient normalized value.

13. The device according to claim 11, wherein the gender prediction loss value or the age prediction loss value is a normalized value based on logarithmic transformation.

14. The device according to claim 9, wherein the communication unit is further configured to receive a recording paper-based ECG image,

the processor is further configured to
extract a line graph by applying an image processing technique from the recording paper-based ECG image, and
converting the extracted ECG signal into data.

15. The device according to claim 14, wherein the processor is further configured to correct distortion of the recording paper-based ECG image using object detection.

1000

KIM OO

FEMALE/
61 YEARS OLD

100

300

200

# FIG. 1

100

FIG. 2A

300

310 — COMMUNICATION INTERFACE

311 — WIRED COMMUNICATION PORT

312 — WIRELESS CIRCUIT

330 — I/O INTERFACE

340 — PROCESSOR

MEMORY — 320

OPERATING SYSTEM — 321

COMMUNICATION MODULE — 322

USER INTERFACE MODULE — 323

APPLICATION — 324

# FIG. 2B

```
           ┌─────────┐
           │  START  │
           └────┬────┘
                │
                ▼
 ┌───────────────────────────────────┐
 │     RECEIVE M-MODE ULTRASOUND      │──── S310
 │         IMAGE OF SUBJECT           │
 └─────────────────┬─────────────────┘
                   │
                   ▼
 ┌───────────────────────────────────┐
 │   PREDICT GENDER AND AGE FOR SUBJECT │
 │   BASED ON RECEIVED ECG DATA USING   │──── S320
 │         PREDICTION MODEL             │
 └─────────────────┬─────────────────┘
                   │
                   ▼
             ┌─────────┐
             │   END   │
             └─────────┘
```

# FIG. 3A

RECEIVE RECORDING PAPER-BASED ECG DATA — S332

EXTRACT LINE GRAPH BY APPLYING
IMAGE PROCESSING TECHNIQUE — S334

# FIG. 3B

# FIG. 3C

414

Shared layers:

- 17×17 conv
- BatchNorm
- Residual block 1
- Residual block 2
- Residual block 2
- Residual block 2
- Residual block 2

4202

420

FC 1 | FC 2

424 — 1 Outputs

2 Outputs — 422

## FIG. 3D

```
┌─────────────────────┐          ┌─────────────────────┐
│  Residual block 1   │          │  Residual block 2   │
└─────────────────────┘          └─────────────────────┘
┌─────────────────────┐          ┌─────────────────────┐
│     17×17 conv      │          │     BatchNorm       │
├─────────────────────┤          ├─────────────────────┤              ┌──────────────────┐
│     BatchNorm       │          │       ReLU          │              │     Max Pool     │
├─────────────────────┤          ├─────────────────────┤              └──────────────────┘
│       ReLU          │          │      Dropout        │
├─────────────────────┤          ├─────────────────────┤              ┌──────────────────┐
│      Dropout        │          │     17×17 conv      │              │    17×17 conv    │
├─────────────────────┤          ├─────────────────────┤              └──────────────────┘
│     17×17 conv      │          │     BatchNorm       │
└─────────────────────┘          ├─────────────────────┤
          ⊕                      │       ReLU          │
                                 ├─────────────────────┤
                                 │      Dropout        │
                                 ├─────────────────────┤
                                 │     17×17 conv      │
                                 └─────────────────────┘
                                           ⊕

┌─────────────────────┐          ┌─────────────────────┐
│        FC 1         │          │        FC 2         │
└─────────────────────┘          └─────────────────────┘
┌─────────────────────┐          ┌─────────────────────┐
│      Flatten        │          │      Flatten        │
├─────────────────────┤          ├─────────────────────┤
│      FC 512         │          │       FC 64         │
├─────────────────────┤          ├─────────────────────┤
│       ReLU          │          │       ReLU          │
└─────────────────────┘          └─────────────────────┘
```

# FIG. 3E

$$\omega_{age} = L_{age}/(L_{age} + L_{gender}) \qquad \omega_{gender} = L_{gender}/(L_{age} + L_{gender})$$

$$L_{total} \quad = N_{grad} \times \omega_{age} \times L_{age} \qquad + N_{grad} \times \omega_{gender} \times L_{gender}$$

$$L_{age} = \log(L_{age} + 1) \qquad L_{gender} = \log(L_{gender} + 1)$$

$$\nabla L_{grad}$$

| Age prediction task | Gender prediction task |

$$L_{grad}$$

| Shared layers | 4202

414

# FIG. 3F

FIG. 4A

FIG. 4B

FIG. 4C

FIG. 4D

Europäisches Patentamt

European Patent Office

Office européen des brevets

## EUROPEAN SEARCH REPORT

**Application Number**

EP 25 17 4682

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2021/361217 A1 (ATTIA ITZHAK ZACHI [US] ET AL) 25 November 2021 (2021-11-25) * paragraphs [0004] - [0007], [0050] - [0053] * | 1-15 | INV. G16H50/20 A61B5/00 G06N3/08 |
| A | US 2023/089991 A1 (ATTIA ITZHAK ZACHI [US] ET AL) 23 March 2023 (2023-03-23) * paragraphs [0104] - [0112] * | 1-15 | |
| Y | Crawshaw Michael: "Multi-Task Learning with Deep Neural Networks: A Survey", arXiv.org, 1 September 2020 (2020-09-01), XP093293749, Retrieved from the Internet: URL:https://arxiv.org/pdf/2009.09796 * pages 1,19-26 * | 1-15 | |
| A | ADRIAN BENTON ET AL: "Multi-Task Learning for Mental Health using Social Media Text", ARXIV.ORG, CORNELL UNIVERSITY LIBRARY, 201 OLIN LIBRARY CORNELL UNIVERSITY ITHACA, NY 14853, 10 December 2017 (2017-12-10), XP080841583, * pages 1-6 * | 1-15 | TECHNICAL FIELDS SEARCHED (IPC) G16H G06N A61B |
| A | WEI-HONG LI ET AL: "Knowledge Distillation for Multi-task Learning", ARXIV.ORG, CORNELL UNIVERSITY LIBRARY, 201 OLIN LIBRARY CORNELL UNIVERSITY ITHACA, NY 14853, 24 September 2020 (2020-09-24), XP081769949, * pages 1-6 * | 1-15 | |

-/--

The present search report has been drawn up for all claims

| Place of search | Date of completion of the search | Examiner |
|---|---|---|
| Munich | 8 September 2025 | Rivera Pons, Carlos |

EPO FORM 1503 03.82 (P04C01)

**page 1 of 2**

## EUROPEAN SEARCH REPORT

**Application Number**

EP 25 17 4682

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SIMON VANDENHENDE ET AL: "Multi-Task Learning for Dense Prediction Tasks: A Survey", ARXIV.ORG, CORNELL UNIVERSITY LIBRARY, 201 OLIN LIBRARY CORNELL UNIVERSITY ITHACA, NY 14853, 16 September 2020 (2020-09-16), XP081764699, * pages 1-17 * ----- | 1-15 | |
| | | | **TECHNICAL FIELDS SEARCHED (IPC)** |

The present search report has been drawn up for all claims

| Place of search | Date of completion of the search | Examiner |
|---|---|---|
| Munich | 8 September 2025 | Rivera Pons, Carlos |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

    .........................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 17 4682

This annex lists the patent family members relating to the patent documents cited in the above-mentioned European search report.
The members are as contained in the European Patent Office EDP file on
The European Patent Office is in no way liable for these particulars which are merely given for the purpose of information.

08-09-2025

| Patent document cited in search report | Publication date | Patent family member(s) | | Publication date |
|---|---|---|---|---|
| US 2021361217 A1 | 25-11-2021 | US | 2021361217 A1 | 25-11-2021 |
| | | WO | 2019140217 A1 | 18-07-2019 |
| US 2023089991 A1 | 23-03-2023 | AU | 2018346412 A1 | 30-04-2020 |
| | | CA | 3078519 A1 | 11-04-2019 |
| | | CN | 111432720 A | 17-07-2020 |
| | | EP | 3691524 A1 | 12-08-2020 |
| | | JP | 7262452 B2 | 21-04-2023 |
| | | JP | 7636461 B2 | 26-02-2025 |
| | | JP | 2020536629 A | 17-12-2020 |
| | | JP | 2023089112 A | 27-06-2023 |
| | | JP | 2025087714 A | 10-06-2025 |
| | | US | 2020397313 A1 | 24-12-2020 |
| | | US | 2023089991 A1 | 23-03-2023 |
| | | US | 2024081653 A1 | 14-03-2024 |
| | | US | 2025000371 A1 | 02-01-2025 |
| | | WO | 2019070978 A1 | 11-04-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240061200
- KR 1020250035870